# EUROPÄISCHE PATENTSCHRIFT

(11) **EP 3 590 576 B1**
(45) Veröffentlichungstag und Bekanntmachung des Hinweises auf die Patenterteilung: **02.09.2026**
(21) Anmeldenummer: 18182196.8
(22) Anmeldetag: 06.07.2018
(51) Int. Cl.: A61N 1/375, A61N 1/362, A61N 1/05

(54) **HEADER MIT RÖNTGENKENNUNG**
HEADER WITH X-RAY DETECTION
EN-TÊTE À IDENTIFICATION PAR RAYONS X

(43) Veröffentlichungstag der Anmeldung: 08.01.2020
(73) Patentinhaber: BIOTRONIK SE & Co. KG, 12359 Berlin (DE)
(72) Erfinder: HENSCHEL, Martin, 13125 Berlin (DE); RUSCHEL, Marina, 12679 Berlin (DE)
(74) Vertreter: Biotronik Corporate Services SE

(56) Entgegenhaltungen:
- EP-A1- 3 238 778
- US-A1- 2009 093 855
- US-A1- 2012 065 503
- US-A1- 2014 058 494

## Beschreibung

Die Offenbarung betrifft einen Header für ein Implantat mit einer Röntgenkennung.

### Hintergrund

Implantate weisen in der Regel eine Röntgenkennung auf. Hiermit werden Informationen zu dem Implantat bereitgestellt, beispielsweise der Hersteller des Implantats. Ein Anwender (z. B. ein Arzt) kann anhand der Herstellerinformation passendes Zubehör zu dem Implantat auswählen, beispielsweise ein kompatibles Programmiergerät.

Zunehmende Packungsdichte der Elektronik und Verkleinerung der Produkte machen es jedoch schwierig, einen Einbauplatz für die Röntgenerkennung im Implantat zu finden, der unter Röntgenlicht auch eindeutig zu erkennen ist.

Für das Anbringen der Röntgenkennung sind verschiedene Möglichkeiten bekannt.

Das Dokument EP 3 238 778 A1 offenbart ein Implantat mit einem Gehäuse und einem Header. Eine Röntgenkennung ist auf einer Leiterplatte angebracht, wobei die Leiterplatte in dem Header angeordnet ist.

Das Dokument US 7,912,549 B2 offenbart ein Implantat mit einem Header. In dem Header ist eine Kavität gebildet, welche eine Röntgenkennung aufnimmt.

Das Dokument 9,808,617 B2 offenbart einen anschlusslosen Herzschrittmacher (leadless pacemaker), wobei eine Röntgenkennung auf dem Gehäuse des Schrittmachers aufgebracht ist.

Gemäß dem Dokument US 2017/0001021 A1 ist eine Röntgenkennung als separates Bauteil mit einer Halterung in dem Header angeordnet oder auf den Header gedruckt.

Alle bekannten Verfahren haben gemeinsam, dass zusätzliche Bauteile für die Röntgenkennung erforderlich sind und/oder zusätzliche Arbeitsschritte zum Erzeugen der Röntgenkennung ausgeführt werden müssen.

### Zusammenfassung

Aufgabe ist es, verbesserte Technologien zum Anbringen einer Röntgenkennung anzugeben.

Es sind eine Elektrodenanschlussvorrichtung nach Anspruch 1 und ein Implantat nach Anspruch 10 offenbart. Weitere Ausführungsformen sind Gegenstand von abhängigen Ansprüchen.

Nach einem Aspekt ist eine Elektrodenanschlussvorrichtung für ein Implantat bereitgestellt, wobei eine Röntgenkennung zur Identifizierung des Herstellers des Implantats in ein metallisches Leitungsband integriert ist.

Die Elektrodenanschlussvorrichtung kann auch Anschlusskopf oder Header genannt werden.

Die Röntgenkennung kann beispielsweise als von dem Leitungsband abstehendes Element realisiert sein. Die Röntgenkennung weist Informationen zu dem Hersteller auf und kann weiter einer Geräteart (z. B. Herzschrittmacher) und/oder eine Seriennummer des Implantats aufweisen.

Die Röntgenkennung kann ein grafisches Symbol aufweisen, beispielsweise ein Firmen-Logo. Die Röntgenkennung kann ein alphanumerisches Zeichen aufweisen. Es können auch mehrere alphanumerische Zeichen vorgesehen sein. Die Röntgenkennung kann eine Kombination von einem oder mehreren grafischen Symbolen und einem oder mehreren alphanumerischen Zeichen aufweisen. Hierdurch sind verschiedene Informationen darstellbar.

Gemäß einem weiteren Aspekt ist ein Implantat mit einer Elektrodenanschlussvorrichtung gemäß den hier offenbarten Merkmalen bereitgestellt.

Die Elektrodenanschlussvorrichtung kann eine Aufnahmeeinrichtung für einen Stecker aufweisen. Die Aufnahmeeinrichtung kann mit einem Leiter verbunden sein, wobei der Leiter mit einer Durchführung verbindbar ist. Die Röntgenkennung kann in den Leiter integriert sein.

Die Elektrodenanschlussvorrichtung kann eine weitere Aufnahmeeinrichtung für einen weiteren Stecker aufweisen. Die weitere Aufnahmeeinrichtung kann mit einem weiteren Leiter verbunden sein, wobei der weitere Leiter mit der Durchführung verbindbar ist. Die Röntgenkennung kann in den weiteren Leiter integriert sein.

Die Elektrodenanschlussvorrichtung kann eine Antenne aufweisen. Die Röntgenkennung kann in die Antenne integriert sein. Die Antenne kann einen Antennenkörper aufweisen, der beispielsweise eine mäandrierende Form haben kann. Die Antenne kann des Weiteren einen Antennenanschluss aufweisen, der mit einer Durchführung verbindbar ist. Die Röntgenkennung kann in den Antennenkörper und/oder in den Antennenanschluss integriert sein.

In einer Ausführungsform weist die Elektrodenanschlussvorrichtung eine Aufnahmeeinrichtung für einen Stecker mit einem Leiter sowie eine Antenne auf. Die Röntgenkennung kann in den Leiter und/oder in die Antenne integriert sein.

Das Implantat kann ein Gehäuse aufweisen, auf welchem die Elektrodenanschlussvorrichtung angeordnet ist. Eine elektrische Verbindung zwischen Komponenten in dem Gehäuse (z. B. einem Elektronikmodul) und Komponenten in der Elektrodenanschlussvorrichtung (z. B. Aufnahmeeinrichtung für einen Stecker oder Antenne) kann mittels einer Durchführung bereitgestellt sein. Die Durchführung kann einen oder mehrere Steckkontakte (z.B. Stifte) zum Anschluss des Leiters (der Leiter) und/oder der Antenne aufweisen. Die Röntgenkennung kann auf einer dem Gehäuse zugewandten Seite (untere Seite) der Elektrodenanschlussvorrichtung gebildet sein. Alternativ kann die Röntgenkennung auf einer dem Gehäuse abgewandten Seite (obere Seite) oder an einer rechten oder linken Seite der Elektrodenanschlussvorrichtung gebildet sein.

Die Elektrodenanschlussvorrichtung kann ein Header für einen implantierbaren Herzschrittmacher oder einen implantierbaren Kardioverter-Defibrillator (ICD - implantable cardioverter-defibrillator) sein. In diesem Fall dient die Elektrodenanschlussvorrichtung zur elektrischen Verbindung von einer oder mehreren Elektrodenleitungen mit dem Implantat.

Das Leitungsband mit der Röntgenkennung kann mit verschiedenen Fertigungsverfahren hergestellt werden, z. B. Erodieren, Stanzen oder additive Fertigungsverfahren wie Lasersintern. Die zusätzliche Kontur der Röntgenerkennung erhöht den Fertigungsaufwand des Leitungsbands (des Leiters/der Antenne) nur unwesentlich, beim Stanzen überhaupt nicht.

Es können mehrere Röntgenkennungen vorgesehen sein. Die mehreren Röntgenkennungen können am selben Leitungsband gebildet sein. Die Aufnahmeeinrichtung kann mehrere Leiter aufweisen (z. B. zwei, drei oder vier Leiter). Jeweils eine Röntgenkennung kann an einem der mehreren Leiter gebildet sein.

Das Leitungsband (z. B. der Leiter oder die Antenne) kann aus einem biokompatiblen Metall bestehen, beispielsweise aus Niob.

Das Leitungsband und die Röntgenkennung können eine einheitliche Dicke haben. Es kann vorgesehen sein, dass das Leitungsband und/oder die Röntgenkennung eine Dicke von wenigstens 50 µm haben. Hierdurch ist eine Sichtbarkeit bei Röntgenstrahlung gegeben. Bevorzugt haben das Leitungsband und/oder die Röntgenkennung eine Dicke zwischen 100 µm und 200 µm.

Zusätzlich zur Röntgenerkennung können auch noch weitere Merkmale in das Leitungsband (die Leiter-/Antennengeometrie) integriert werden, die zum Positionieren, Prüfen und Handling verwendet werden können, z. B. eine Indexbohrung.

### Beschreibung von Ausführungsbeispielen

Im Folgenden werden beispielhafte Ausführungsformen unter Bezugnahme auf Figuren näher erläutert. Es zeigen:
- Fig. 1: ein Ausschnitt eines Implantats mit einem Header, wie er aus dem Stand der Technik bekannt ist,
- Fig. 2: ein Ausschnitt eines Implantats mit einer ersten Ausführungsform eines erfindungsgemäßen Headers und
- Fig. 3: ein Ausschnitt eines Implantats mit einer zweiten Ausführungsform eines erfindungsgemäßen Headers.

Im Folgenden werden für gleiche Komponenten gleiche Bezugszeichen verwendet.

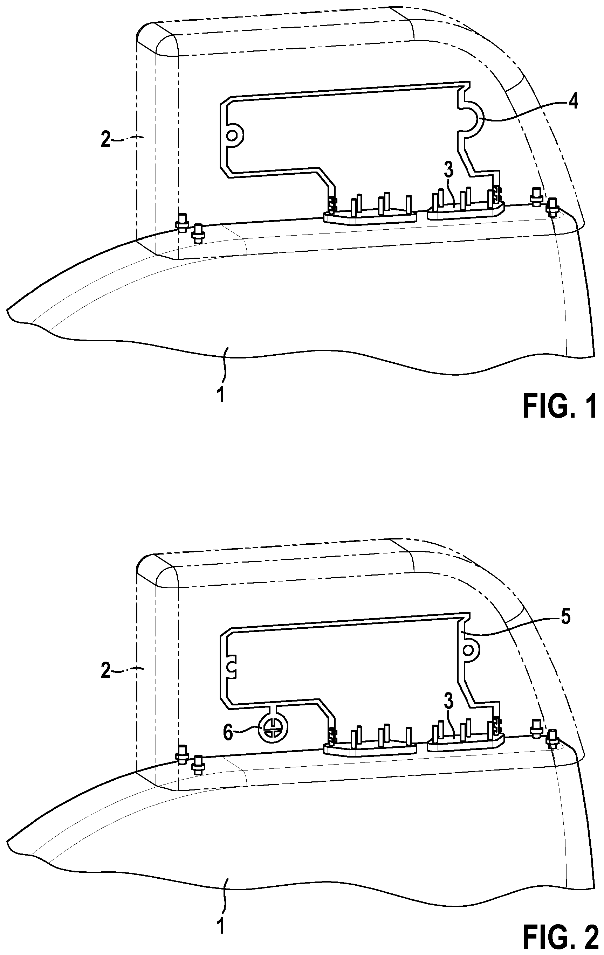

Fig. 1 zeigt ein Implantat mit einem Gehäuse 1 auf dem eine Elektrodenanschlussvorrichtung (Header) 2 angeordnet ist. In der Elektrodenanschlussvorrichtung 2 ist eine herkömmliche Antenne 4 angeordnet. Die Antenne 4 ist mittels einer Durchführung 3 mit elektronischen Komponenten im Gehäuse 1 verbunden.

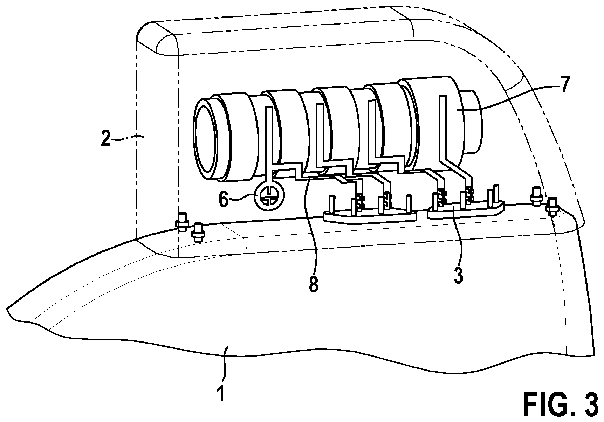

In Fig. 2 ist ein erfindungsgemäße Ausführungsform der Elektrodenanschlussvorrichtung 2 gezeigt. Eine Antenne 5 weist eine integrierte Röntgenkennung 6 auf. Die Röntgenkennung 6 ist als ein von der Antenne 5 abstehendes Element ausgeführt. Die Durchführung 3 weist mehrere Stifte auf, welche zum Anschluss der Antenne 5 und weiterer Komponenten (Fig. 3) dienen.

Fig. 3 zeigt eine weitere Ausführungsform einer Elektrodenanschlussvorrichtung 2 gemäß der Erfindung. In der Elektrodenanschlussvorrichtung 2 ist eine Aufnahmeeinrichtung 7 für einen Stecker angeordnet. Die Aufnahmeeinrichtung 7 ist mittels eines Leiters 8 mit der Durchführung 3 verbunden. Die Röntgenkennung 6 ist als integraler Bestandteil des Leiters 8 gebildet und steht von diesem ab.

Die hier offenbarte Lehre bietet die Möglichkeit, die Röntgenkennung in einen vorhandenen Fertigungsprozess zu integrieren. Kosten für eine zusätzliche Komponente werden eingespart. Es kann auf einen zusätzlichen Klebeschritt oder Schweißprozess zum Befestigen der Röntgenerkennung verzichtet werden.

Die in der Beschreibung, den Ansprüchen und den Figuren offenbarten Merkmale können für die Verwirklichung von Ausführungsformen sowohl einzeln als auch in beliebiger Kombination miteinander relevant sein.

### Bezugszeichenliste:

- 1: Gehäuse
- 2: Elektrodenanschlussvorrichtung
- 3: Durchführung
- 4: Antenne ohne Röntgenkennung
- 5: Antenne mit Röntgenkennung
- 6: Röntgenkennung
- 7: Aufnahmeeinrichtung für Stecker
- 8: Leiter

## Patentansprüche

1. Elektrodenanschlussvorrichtung für ein Implantat, umfassend eine Röntgenkennung (6) zur Identifizierung des Herstellers des Implantats und ein metallisches Leitungsband (5, 8),
**dadurch gekennzeichnet,**
**dass** die Röntgenkennung (6) in das metallisches Leitungsband (5, 8) integriert ist.

2. Elektrodenanschlussvorrichtung nach Anspruch 1, aufweisend eine Aufnahmeeinrichtung (7) für einen Stecker, wobei das metallische Leitungsband als ein Leiter (8) ausgebildet ist und die Aufnahmeeinrichtung (7) mit dem Leiter (8) verbunden ist, wobei der Leiter (8) mit einer Durchführung (3) verbindbar ist und wobei die Röntgenkennung (6) in den Leiter (8) integriert ist.

3. Elektrodenanschlussvorrichtung nach Anspruch 1 oder 2, wobei das metallische Leitungsband als eine Antenne (5) ausgebildet ist, und wobei die Röntgenkennung (6) in die Antenne (5) integriert ist.

4. Elektrodenanschlussvorrichtung nach einem der vorangehenden Ansprüche, wobei das metallische Leitungsband (5, 8) aus einem biokompatiblen Metall besteht.

5. Elektrodenanschlussvorrichtung nach Anspruch 4, wobei das metallische Leitungsband (5, 8) aus Niob besteht.

6. Elektrodenanschlussvorrichtung nach einem der vorangehenden Ansprüche, wobei das metallische Leitungsband (5, 8) und die Röntgenkennung (6) eine einheitliche Dicke haben.

7. Elektrodenanschlussvorrichtung nach einem der vorangehenden Ansprüche, wobei das metallische Leitungsband (5, 8) und/oder die Röntgenkennung (6) eine Dicke von wenigstens 50 µm haben.

8. Elektrodenanschlussvorrichtung nach einem der vorangehenden Ansprüche, wobei die Röntgenkennung (6) ein grafisches Symbol aufweist.

9. Elektrodenanschlussvorrichtung nach einem der vorangehenden Ansprüche, wobei die Röntgenkennung (6) ein alphanumerisches Zeichen aufweist.

10. Implantat mit einer Elektrodenanschlussvorrichtung nach einem der vorangehenden Ansprüche.

## Claims

1. An electrode connection device for an implant, comprising a radiographic marker (6) for identifying the manufacturer of the implant and a metal conducting ribbon (5, 8),
**characterized in that**
the radiographic marker (6) is integrated into the metal conducting ribbon (5, 8).

2. The electrode connection device according to claim 1, comprising a receiving device (7) for a connector, wherein the metal conducting ribbon is designed as a conductor (8), and the receiving device (7) is connected to the conductor (8), the conductor (8) being connectible to a feedthrough (3), and the radiographic marker (6) being integrated into the conductor (8).

3. The electrode connection device according to claim 1 or 2, wherein the metal conducting ribbon is designed as an antenna (5), and the radiographic marker (6) is integrated into the antenna (5).

4. The electrode connection device according to any one of the preceding claims,
wherein the metal conducting ribbon (5, 8) is made of a biocompatible metal.

5. The electrode connection device according to claim 4, wherein the metal conducting ribbon (5, 8) is made of niobium.

6. The electrode connection device according to any one of the preceding claims,
wherein the metal conducting ribbon (5, 8) and the radiographic marker (6) have a uniform thickness.

7. The electrode connection device according to any one of the preceding claims,
wherein the metal conducting ribbon (5, 8) and/or the radiographic marker (6) have a thickness of at least 50 µm.

8. The electrode connection device according to any one of the preceding claims,
wherein the radiographic marker (6) includes a graphic symbol.

9. The electrode connection device according to any one of the preceding claims,
wherein the radiographic marker (6) includes an alphanumeric code.

10. An implant comprising an electrode connection device according to any one of the preceding claims.

## Revendications

1. Dispositif de connexion d'électrodes pour un implant, comprenant un marqueur radiographique (6) pour identifier le fabricant de l'implant et un ruban conducteur métallique (5, 8),
**caractérisé en ce que**
le marqueur radiographique (6) est intégré au ruban conducteur métallique (5, 8).

2. Dispositif de connexion d'électrodes selon la revendication 1, comprenant un dispositif récepteur (7) pour un connecteur, dans lequel le ruban conducteur métallique est conçu en tant que conducteur (8), et le dispositif de réception (7) est connecté au conducteur (8), le conducteur (8) pouvant être connecté à une connexion d'interface (3), et le marqueur radiographique (6) étant intégré dans le conducteur (8).

3. Dispositif de connexion d'électrodes selon la revendication 1 ou 2, dans lequel le ruban conducteur métallique est conçu en tant qu'antenne (5), et le marqueur radiographique (6) est intégré dans l'antenne (5).

4. Dispositif de connexion d'électrodes selon l'une quelconque des revendications précédentes, dans lequel le ruban conducteur métallique (5, 8) est fabriqué en un métal biocompatible.

5. Dispositif de connexion d'électrodes selon la revendication 4, dans lequel le ruban conducteur métallique (5, 8) est fabriqué en niobium.

6. Dispositif de connexion d'électrodes selon l'une quelconque des revendications précédentes, dans lequel le ruban conducteur métallique (5, 8) et le marqueur radiographique (6) ont une épaisseur uniforme.

7. Dispositif de connexion d'électrodes selon l'une quelconque des revendications précédentes, dans lequel le ruban conducteur métallique (5, 8) et/ou le marqueur radiographique (6) ont une épaisseur d'au moins 50 µm.

8. Dispositif de connexion d'électrodes selon l'une quelconque des revendications précédentes, dans lequel le marqueur radiographique (6) inclut un symbole graphique.

9. Dispositif de connexion d'électrodes selon l'une quelconque des revendications précédentes, dans lequel le marqueur radiographique (6) inclut un code alphanumérique.

10. Implant comprenant un dispositif de connexion d'électrodes selon l'une quelconque des revendications précédentes.
